# EUROPEAN PATENT SPECIFICATION

(11) **EP 4 037 648 B1**
(45) Date of publication and mention of the grant of the patent: **06.11.2024**
(21) Application number: 20871260.4
(22) Date of filing: 30.09.2020
(51) Int. Cl.: A61K 8/44, A61K 8/21, A61K 8/19, A61K 8/25, A61Q 11/00

(54) **DENTIFRICE COMPOSITIONS COMPRISING BICARBONATE SALT AND NEUTRAL AMINO ACID**
ZAHNPASTENZUSAMMENSETZUNGEN MIT BICARBONATSALZ UND NEUTRALER AMINOSÄURE
COMPOSITIONS DE DENTIFRICE COMPRENANT UN SEL DE BICARBONATE ET UN ACIDE AMINÉ NEUTRE

(30) Priority: 30.09.2019 WO PCT/CN2019/109404
(43) Date of publication of application: 10.08.2022
(73) Proprietor: The Procter & Gamble Company, Cincinnati, OH 45202 (US)
(72) Inventor: STRAND, Ross, Singapore 138547 (SG); SHI, Yunming, Beijing 101312 (CN); GAO, Zhaoxin, Beijing 101312 (CN); LI, Xinxin, Beijing 101312 (CN)
(74) Representative: P&G Patent Germany
(86) International application number: PCT/CN2020/119208
(87) International publication number: WO 2021/063384

(56) References cited:
- EP-A1- 2 275 084
- EP-A1- 2 275 084
- WO-A1-2005/092277
- WO-A1-2015/094836
- WO-A1-2015/094836
- CA-A1- 2 170 532
- CA-A1- 2 170 532
- CN-A- 106 937 921
- CN-A- 106 937 921
- US-A1- 2020 146 958
- US-A1- 2020 146 959
- DATABASE GNPD [online] MINTEL; 19 July 2002 (2002-07-19), ANONYMOUS: "Signal Aliento Fresco Toothpaste", XP055871930, retrieved from https://www.gnpd.com/sinatra/recordpage/158812/ Database accession no. 158812
- DATABASE GNPD [online] MINTEL; 5 April 2013 (2013-04-05), ANONYMOUS: "2 in 1 Power Fresh Mouth Spray", XP055871926, retrieved from https://www.gnpd.com/sinatra/recordpage/2037646/ Database accession no. 2037646
- DATABASE GNPD [online] MINTEL; 14 May 2007 (2007-05-14), ANONYMOUS: "Toothpaste Repackaging", XP055871929, retrieved from https://www.gnpd.com/sinatra/recordpage/702070/ Database accession no. 702070
- DATABASE GNPD [online] MINTEL; 26 September 2012 (2012-09-26), ANONYMOUS: "Toothpaste", XP055871928, retrieved from https://www.gnpd.com/sinatra/recordpage/1892762/ Database accession no. 1892762
- DATABASE GNPD [online] MINTEL; 14 May 2007 (2007-05-14), ANONYMOUS: "Toothpaste Repackaging", XP055871929, retrieved from https://www.gnpd.com/sinatra/recordpage/702070/ Database accession no. 702070

## Description

### FIELD OF THE INVENTION

The present invention relates to dentifrice compositions comprising bicarbonate salt and neutral amino acid. The dentifrice compositions have improved efficacy to help inhibit biofilm formation or help disrupt biofilm.

### BACKGROUND OF THE INVENTION

Dental plaque (also known as dental biofilm) is a sticky, colorless deposit of bacteria that is constantly forming on the tooth surface. Dental plaque is generally made up of bacteria and extracellular polymer substances (so called "EPS"). EPS are biopolymers of microbial origin in which biofilm microorganisms are embedded. J. Bacteriol. 2007, 189(22):7945*.* Saliva, food and fluids combine to produce these deposits that collect where the teeth and gums meet. Plaque buildup is the primary factor in poor oral health that can lead to caries and periodontal (gum) disease, including gingivitis. One-way dentifrice compositions help prevent and control plaque is by leveraging anti-bacterial agents; however, the disadvantage and formulation challenge is the unintended reactivity of anti-bacterial agents with formulation ingredients. This may include oxidative degradation, hydrolysis, adsorption or precipitation of oxy-hydroxide species, any of which can impact the bio-availability of the anti-bacterial agent. There is a continuing consumer need to provide such formulations that help prevent plaque formation on teeth and/or minimize the use of antimicrobial agents.

One solution to help inhibit biofilm formation or help disrupt biofilm is the use of baking soda (i.e., sodium bicarbonate). Baking soda mechanism of action against biofilm is likely at least two-fold. Baking soda can displace calcium ions so as to help disrupt or reduce the biofilm. Calcium ions act as a "glue" or "scaffold" of EPS components of dental biofilm. Baking soda may also act as an abrasive to aid in the physical removal of oral biofilm. Some levels of baking soda in dentifrice are reported at over 30 wt.%, and sometimes higher than 60 wt.%. However, some users report an unpleasant taste experience (attributable to the relatively high level of baking soda). The use of high-level baking soda compositions brings stability challenges around decomposition of the baking soda. An approach used to overcome such stability challenges has been to formulate baking soda composition in an anhydrous or low water compositions. However, this approach can lead to poor rheology properties and impairs the dispersion and delivery. Furthermore, dental plaque is particularly problematic in developing markets. And thus, dentifrice solutions are best cost effective (e.g., containing a relatively high level of water). Accordingly, there is a need for baking soda containing aqueous dentifrice compositions, maximizing the bicarbonate salt associated therapeutic benefits while minimizing the level of bicarbonate salt (e.g., < 60 wt.%) as to help with the flavor profile of the composition and improved dispersion and delivery. There is also a need for a composition that is cost effective for developing markets where arguably the need for such compositions are greatest.

CA2170532 A1 shows a toothpaste comprising 10-65% sodium bicarbonate in an aqueous solution of sorbitol, and also comprising fluoride.

### SUMMARY OF THE INVENTION

The present invention relates to a dentifrice composition comprising: (a) from 15% to 55%, by weight of the composition, of water; (b) from 20% to 40%, by weight of the composition, of bicarbonate salt; (c) from 0.01% to 10%, by weight of the composition, of neutral amino acid; and (d) from 0.0025% to 2%, by weight of the composition, of a fluoride ion source.

One advantage of the present invention is the significant disruption and destabilization of the dental biofilm architecture achieved by a dentifrice composition comprising the combination of bicarbonate salt (e.g. sodium bicarbonate) and neutral amino acid (e.g. glycine). To this end, it is further surprising that the effect of the two components, in themselves, do not act as cidal or have an inhibitory impact on bacteria.

Further, there are commercial dentifrice compositions containing over 65% sodium bicarbonate, which claim to provide a clinical benefit in treating gingivitis. However, users report an unpleasant sensory experience (e.g. taste, foaming, dispersion) associated at this higher a level of sodium bicarbonate. Another aspect of this invention is thus combining bicarbonate salt with neutral amino acid to provide a dentifrice composition that allows lowering the level of bicarbonate salt, while still destabilizing the biofilm and providing an improved sensorial experience.

Yet another aspect of this invention is the formulation of aqueous dentifrice containing relatively high content of both baking soda and water that has a good chemical and physical stability over the product shelf life, allowing for an improved dispersion and delivery that maximizes the bicarbonate salt associated therapeutic benefits.

Yet another aspect of the invention provides a method of treating dental biofilm comprising the step of brushing teeth with a composition of the present invention.

Yet still another aspect of the invention provides a method preventing or mitigating plaque formation on tooth enamel comprising the step of brushing teeth with a dentifrice composition of the present invention.

### BRIEF DESCRIPTION OF THE DRAWINGS

Figure 1 is a perspective view of an oral splint with Hydroxyapatite ("HA') disks attached thereto.
Figure 2 is a perspective view of the HA disk having grooves therein.
Figure 3 is a schematic of a cross sectional view of the groove with biofilm therein.

### DETAILED DESCRIPTION OF THE INVENTION

### Definitions

The term "comprising" as used herein means that steps and ingredients other than those specifically mentioned can be added. This term encompasses the terms "consisting of" and "consisting essentially of." The compositions of the present invention can comprise, consist of, and consist essentially of the essential elements and limitations of the invention described herein, as well as any of the additional or optional ingredients, components, steps, or limitations described herein.

The term "dentifrice" as used herein means paste, gel, powder, tablets, or liquid formulations, unless otherwise specified, that are used to clean the surfaces of the oral cavity. Preferably the dentifrice compositions of the present invention are single phase compositions. One example of a dentifrice is toothpaste (for brushing teeth). The term "teeth" as used herein refers to natural teeth as well as artificial teeth or dental prosthesis.

All percentages, parts and ratios are based upon the total weight of the compositions of the present invention, unless otherwise specified. All such weights as they pertain to listed ingredients are based on the active level and, therefore do not include solvents or by-products that may be included in commercially available materials, unless otherwise specified. The term "weight percent" may be denoted as "wt.%" herein. All molecular weights as used herein are weight average molecular weights expressed as grams/mole, unless otherwise specified.

As used herein, the articles including "a" and "an" when used in a claim, are understood to mean one or more of what is claimed or described.

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

### Water

The dentifrice compositions of the present invention comprise herein from 15% to 55%, preferably from 15% to 45%, preferably from 20% to 35%. by weight of the composition, of water. The dentifrice composition can comprise from 15% to 55%, preferably from 20% to 45%, preferably from 25% to 35%, by weight of the composition, of water. The water may be added to the formulation and/or may come into the composition from the inclusion of other ingredients provided in aqueous solution. Preferably the water is USP water.

### Bicarbonate Salt

The compositions of the present invention comprise from 20% to 40% by weight of the composition, of bicarbonate salt. Preferably, the bicarbonate salt is selected from sodium bicarbonate, calcium bicarbonate, or mixtures thereof; more preferably sodium bicarbonate.

### Neutral Amino acids

The dentifrice compositions of the present invention comprise a neutral amino acid.

The term "neutral amino acids" as used herein include not only naturally occurring neutral amino acids, such as alanine, asparagine, cysteine, glutamine, glycine, isoleucine, leucine, methionine, phenylalanine, proline, serine, threonine, tryptophan, tyrosine, valine, but also biologically acceptable amino acid which has an isoelectric point in range of pH 5.0 to 7.0. The biologically preferred acceptable neutral amino acid has a single amino group and carboxyl group in the molecule or a functional derivative hereof, such as functional derivatives having an altered side chain albeit similar or substantially similar physio chemical properties. In a further embodiment the amino acid would be at minimum partially water soluble and provide a pH of less than 7 in an aqueous solution of 1g/1000ml at 25°C.

Accordingly, neutral amino acids suitable for use in the invention include, but are not limited to, alanine, aminobutyrate, asparagine, cysteine, cystine, glutamine, glycine, hydroxyproline, isoleucine, leucine, methionine, phenylalanine, proline, serine, taurine, threonine, tryptophan, tyrosine, valine, salts thereof, or mixtures thereof. Preferably, neutral amino acids used in the composition of the present invention may include asparagine, glutamine, glycine, salts thereof, or mixtures thereof. The neutral amino acids may have an isoelectric point of 5.0, or 5.1, or 5.2, or 5.3, or 5.4, or 5.5, or 5.6, or 5.7, or 5.8, or 5.9, or 6.0, or 6.1, or 6.2, or 6.3, or 6.4, or 6.5, or 6.6, or 6.7, or 6.8, or 6.9, or 7.0, in an aqueous solution at 25°C. Preferably, the neutral amino acid is selected from asparagine, glutamine, or glycine, more preferably in its free form. If the neutral amino acid is in its salt form, suitable salts include salts known in the art to be pharmaceutically acceptable salts considered to be physiologically acceptable in the amounts and concentrations provided. The neutral amino acid is present in the amount of from 0.01% to 10%, preferably from 0.05% to 5%, preferably from 0.1% to 3%, preferably from 0.5% to 3%, preferably from 1% to 3%, by weight of the composition. In one aspect, the neutral amino acid is glutamine (or salt thereof). In another aspect, the neutral amino acid is asparagine (or salt thereof). In yet another aspect, the neutral amino acid is glycine (or salt thereof).

It has been surprisingly discovered that, the use of neutral amino acid provides biofilm prevention or reduction benefits in compositions containing bicarbonate salt (e.g. at reduced levels) while enabling improved taste due to relatively reduced levels of bicarbonate salt.

Furthermore, the introductions of amino acid provide gingival would healing benefit and thus be able to minimize the use of other anti-bleeding agents, for example tranexamic acid, epsilon aminocaproic acid, and p-aminomethylbenzoic acid. In one aspect, the dentifrice composition of the present invention is substantially free of, preferably essentially free of, and more preferably free of, tranexamic acid, epsilon aminocaproic acid, and p-aminomethylbenzoic acid.

In one aspect, the neutral amino acid is uncomplexed. The neutral amino acid is not complexed with zinc.

### Fluoride ion source

The compositions include an effective amount of an anti-caries agent. In one aspect, the anti-caries agent is a fluoride ion source. The fluoride ion may be present in an amount sufficient to give a fluoride ion concentration in the composition at 25°C, and/or in one embodiment can be used at levels of from 0.0025% to 5% by weight of the composition, preferably from 0.005% to 2.0%, by weight of the composition, to provide anti-caries effectiveness. Representative fluoride ion sources include: stannous fluoride, sodium fluoride, potassium fluoride, amine fluoride, sodium monofluorophosphate, zinc fluoride, or mixtures thereof. In one aspect, the dentifrice compositions of the present invention may have a dual fluoride ion source, specifically sodium monofluorophosphate and an alkaline metal fluoride. Without wishing to be bound by theory, such an approach may provide an improvement in mean fluoride uptake.

### Calcium-containing abrasive

The compositions of the present invention can optionally, and in some aspects preferably, comprise from 5% to 50%, preferably from 10% to 50%, by weight of the composition, of a calcium-containing abrasive, wherein preferably the calcium-containing abrasive is selected from the group consisting of calcium carbonate, calcium glycerophosphate, dicalcium phosphate, tricalcium phosphate, calcium orthophosphate, calcium metaphosphate, calcium polyphosphate, calcium oxyapatite, sodium carbonate, and mixtures thereof; wherein more preferably the calcium-containing abrasive is calcium carbonate. Preferably, the composition comprises from 15% to 50%, preferably from 15% to 30%, preferably from 15% to 25%, by weight of the composition, of a calcium-containing abrasive.

Preferably, the calcium-containing abrasive is calcium carbonate. More preferably, the calcium-containing abrasive is selected from the group consisting of fine ground natural chalk, ground calcium carbonate, precipitated calcium carbonate, and combinations thereof. Non-limiting examples of the weight percentages of the calcium-containing abrasive include: 15%, 20%, 25%, or 30%, by weight of the composition, preferably wherein the calcium-containing abrasive is calcium carbonate.

### Silica abrasive

The compositions of the present invention can optionally, and in some aspects preferably, comprise silica abrasive. Silica dental abrasives of various types, such as thickening silica, are preferred because of their unique benefits of exceptional dental cleaning and polishing performance without unduly abrading tooth enamel or dentine. The silica abrasive polishing materials herein, as well as other abrasives, generally have an average particle size ranging between about 0.1 to about 30 microns, and preferably from about 5 to about 15 microns. The abrasive can be precipitated silica or silica gels such as the silica xerogels describe in Pader et al., U.S. Patent 3,538,230, issued Mar. 2, 1970, and DiGiulio, U.S. Patent 3,862,307, issued Jan. 21, 1975. Preferred are the silica xerogels marketed under the trade name "Syloid" by the W.R Grace & Company, Davison Chemical Division. Also preferred are the precipitated silica materials such as those marketed by the J. M. Huber Corporation under the trade name, Zeodent^{®}, particularly the silicas carrying the designation Zeodent^{®} 119, Zeodent^{®} 118, Zeodent^{®} 109 and Zeodent^{®} 129. The types of silica dental abrasives useful in the toothpastes of the present invention are described in more detail in Wason, U.S. Patent 4,340,583, issued July 29, 1982; and in commonly-assigned US Pat Nos. 5,603,920, issued on Feb. 18, 1997; 5,589,160, issued Dec. 31, 1996; 5,658,553, issued Aug. 19, 1997; 5,651,958, issued July 29, 1997, and U.S. Provisional Application Serial No. 60/300766, filed June 25, 2001.

In one aspect, the composition comprises from 0% to 15%, preferably 0.5% to 10%, more preferably 1% to 5%, by weight of the composition, of a silica abrasive In one aspect, the composition is substantially free of silica abrasive.

In one aspect, the composition comprises calcium-containing abrasive and silica abrasive. pH

The pH of the dentifrice composition may be greater than about 7.8, preferably greater than about 8, preferably from about 8 to about 11, preferably from about 8.5 to about 11, preferably about 8.5 to about 10.5, preferably from about 8.5 to about 10. The relatively high pH of the present inventive composition is for fluoride stability. Without wishing to be bound theory, at below pH 8 calcium ion may bind with the fluoride. Thus, it is desirable to have the dentifrice composition have a greater than pH 8.0 to maximize the stability of the fluoride ion source. A method for assessing pH of dentifrice is described is below. For purposes of clarification, although the analytical method describes testing the dentifrice composition when freshly prepared, for purposes of claiming the present invention, the pH may be taken at anytime during the product's reasonable lifecycle (including but not limited to the time the product is purchased from a store and brought to the user's home).

### pH modifying agent

The dentifrice compositions herein may include an effective amount of a pH modifying agent, preferably wherein the pH modifying agent is a pH buffering agent. The pH modifying agents, as used herein, refer to agents that can be used to adjust the pH of the dentifrice compositions to the above-identified pH range. The compositions can comprise from about 0.001% to about 5%, by weight of the composition, of pH modifying agent. The pH modifying agents may include alkali metal hydroxides, ammonium hydroxide, organic ammonium compounds, carbonates, sesquicarbonates, borates, silicates, phosphates, imidazole, and mixtures thereof. Specific pH agents include monosodium phosphate (monobasic sodium phosphate or "MSP"), trisodium phosphate (sodium phosphate tribasic dodecahydrate or "TSP"), sodium benzoate, benzoic acid, sodium hydroxide, potassium hydroxide, alkali metal carbonate salts, sodium carbonate, imidazole, pyrophosphate salts, tripolyphoshpate salts, sodium gluconate, lactic acid, sodium lactate, citric acid, sodium citrate, phosphoric acid. Without wishing to be bound by theory, phosphate may also have calcium ion chelating activity and therefore provide some monofluorophosphate stabilization (in those formulations containing monofluorophosphate).

A method for assessing pH of dentifrice is described. The pH is measured by a pH Meter with Automatic Temperature Compensating (ATC) probe. The pH Meter is capable of reading to 0.001 pH unit. The pH electrode may be selected from one of the following (i) Orion Ross Sure-Flow combination: Glass body - VWR #34104-834/Orion #8172BN or VWR#10010-772/Orion #8172BNWP; Epoxy body - VWR #34104-830/Orion #8165BN or VWR#10010-770/Orion #8165BNWP; Semi-micro, epoxy body - VWR #34104-837/Orion #8175BN or VWR#10010-774/Orion #3175BNWP; or (ii) Orion PerpHect combination:VWR #34104-843/Orion #8203BN semi-micro, glass body; or (iii) suitable equivalent. The automatic temperature compensating probe is Fisher Scientific, Cat #13-620-16.

A 25% by weight slurry of dentifrice is prepared with deionized water, and thereafter is centrifuged for 10 minutes at 15000 rotations-per-minute using a SORVALL RC 28S centrifuge and SS-34 rotor (or equivalent gravitational force, at 24149g force). The pH is assessed in supernatant after one minute or the taking reading is stabilized. After each pH assessment, the electrode is washed with deionized water. Any excess water is wiped with a laboratory grade tissue. When not in issue, the electrode is kept immersed in a pH 7 buffer solution or an appropriate electrode storage solution.

### Humectants

The compositions herein contains relatively low amount, or even be substantially free or free, of humectants. Non-limiting examples of humectant levels, by weight of the dentifrice composition, include no more than about 0.1%, no more than about 0.5%, no more than about 1%, no more than about 5%, no more than about 10%, or 0%. Without wishing to be bound by theory, the presence of humectant (e.g., sorbitol/glyercol) may have a negative role in fluoride uptake in dental plaque in the high water and high carbonate containing dentifrice formulations of the present invention. Reduced levels of sorbitol/glycerol in these dentifrice compositions provide superior fluoride uptake results. Preferably the dentifrice compositions of the present invention comprise from 0% to about 10%, by weight of the composition, of a humectant, wherein the humectant is selected from sorbitol, glycerol, or mixtures thereof; more preferably the composition comprises from 0% to about 7%, preferably 0% to about 5%, by weight of the composition of said humectant. In one aspect, the composition is substantially free of humectant.

The term "humectant," for the purposes of present invention, include edible polyhydric alcohols such as glycerin, sorbitol, xylitol, butylene glycol, propylene glycol, or mixtures thereof. In one aspect, the humectant is a polyol, preferably wherein the polyol is selected from sorbitol, glycerin, or mixtures thereof. In yet another example, the humectant is sorbitol. A potential advantage of having a dentifrice composition that contains low levels of humectant (i.e., at or less than about 2 wt.%), without wishing to be bound by theory, is those dentifrice compositions that are free of humectants such as glyercol or sorbitol may provide better fluoride uptake as compared to those compositions having the high levels of such humectants. In one aspect, the dentifrice compositions of the present invention comprise from 0% to about 2%, preferably 0% to about 1%, more preferably 0% to about 0.5%, by weight of the composition, of glycerin and/or sorbitol. In one aspect, the composition is preferably substantially free of both glycerin and sorbitol.

### Thickening System

The dentifrice compositions of the present invention may comprise a thickening system. Preferably the dentifrice composition comprises from about 0.5% to about 10%, preferably from about 0.8% to about 5%, more preferably from about 1% to about 5%, by weight of the composition, of the thickening system. More preferably the thickening system comprises a thickening polymer, a thickening silica, or mixtures thereof. Yet more preferably, when the thickening system comprises a thickening polymer, the thickening polymer is selected from a carboxymethyl cellulose, a linear sulfated polysaccharide, a natural gum, or mixtures thereof. Yet still more preferably, when the thickening system comprises a thickening polymer, the thickening polymer is selected from the group consisting of: (a) from about 0.01% to about 3% of a carboxymethyl cellulose ("CMC") by weight of the composition, preferably from about 0.1% to about 2.5%, more preferably from about 0.5% to about 1.7%, by weight of the composition, of CMC; (b) from about 0.01% to about 2.5%, preferably from about 0.05 % to about 2%, more preferably from about 0.1% to about 1.5%, by weight of the composition, of a linear sulfated polysaccharide, preferably wherein the linear sulfated polysaccharide is a carrageenan; (c) from about 0.01% to about 3%, preferably from about 0.1% to about 2%, more preferably from about 0.2% to about 1.8%, by weight of the composition, of a natural gum; or (d) mixtures thereof.

Preferably, when the thickening system comprises a thickening silica, the thickening silica is from about 0.01% to about 8%, preferably from about 0.1% to about 5%, preferably about 1% to about 3%, by weight of the composition.

Preferably, the linear sulfated polysaccharide is a carrageenan (also known as carrageenan). Examples of carrageenan include Kappa-carrageenan, Iota-carrageenan, Lambda-carrageenan, or mixtures thereof.

In one aspect the thickening silica is obtained from sodium silicate solution by destabilizing with acid as to yield very fine particles. One commercially available example is ZEODENT^{®} branded silicas from Huber Engineered Materials (e.g., ZEODENT^{®} 103, 124, 113 115, 163, 165, 167).

In one aspect, the CMC is prepared from cellulose by treatment with alkali and monochloro-acetic acid or its sodium salt. Different varieties are commercially characterized by viscosity. One commercially available example is AqualonTM branded CMC from Ashland Special Ingredients (e.g., AqualonTM 7H3SF; AqualonTM 9M3SF AqualonTM TM9A; AqualonTM TM12A).

Preferably, a natural gum is selected from the group consisting of gum karaya, gum arabic (also known as acacia gum), gum tragacanth, xanthan gum, and mixtures thereof. More preferably the natural gum is xanthan gum. Xanthan gum is a polysaccharide secreted by the bacterium Xanthomonas camestris. Generally, xanthan gum is composed of a pentasaccharide repeat units, comprising glucose, mannose, and glucuronic acid in a molar ratio of 2:2:1, respectively. The chemical formula (of the monomer) is C₃₅H₄₉O₂₉. In one aspect, the xanthan gum is from CP Kelco Inc (Okmulgee, US).

### Viscosity

Preferably the dentifrice compositions of the present invention have a viscosity range from about 200,000 centipoise to about 850,000 centipoise ("cP"). A method for assessing viscosity is described. The viscometer is Brookfield^{®} viscometer, Model DV-I Prime with a Brookfield "Helipath" stand. The viscometer is placed on the Helipath stand and leveled via spirit levels. The E spindle is attached, and the viscometer is set to 2.5 RPM. Detach the spindle, zero the viscometer and install the E spindle. Then, lower the spindle until the crosspiece is partially submerged in the paste before starting the measurement. Simultaneously turn on the power switch on the viscometer and the helipath to start rotation of the spindle downward. Set a timer for 48 seconds and turn the timer on at the same time as the motor and helipath. Take a reading after the 48 seconds. The reading is in cP.

### PEG

The compositions of the present invention may optionally comprise polyethylene glycol (PEG), of various weight percentages of the composition as well as various ranges of average molecular weights. In one aspect of the invention, the compositions have from about 0.1% to about 5%, preferably from about 0.5% to about 4%, more preferably from about 1% to about 3%, by weight of the composition, of PEG. In another aspect of the invention, the PEG is one having a range of average molecular weight from about 100 to about 1600, preferably from about 200 to about 1000, preferably from about 400 to about 800, preferably from about 500 to about 700 Daltons. PEG is a water-soluble linear polymer formed by the addition reaction of ethylene oxide to an ethylene glycol equivalent having the general formula is: H-(OCH₂CH₂)ₙ-OH. One supplier of PEG is Dow Chemical Company under the brandname of CARBOWAX^{™}. Without wishing to be bound by theory, having some PEG in the dentifrice composition may help with physical stability.

### Sweetener

The dentifrice compositions herein may include a sweetening agents. These sweetening agents may include saccharin, dextrose, sucrose, lactose, maltose, levulose, aspartame, stevia, sodium cyclamate, brazzein, pentadin, D-tryptophan, dihydrochalcones, acesulfame, sucralose, neotame, and mixtures thereof. Sweetening agents are generally used in oral compositions at levels of from about 0.005% to about 5%, preferably from about 0.01% to about 1%, preferably from about 0.1% to about 0.5%, by weight of the composition.

### Surfactant

The dentifrice compositions herein may include a surfactant. The surfactant may be selected from anionic, nonionic, amphoteric, zwitterionic, cationic surfactants, or mixtures thereof. The composition may include a surfactant at a level of from about 0.01% to about 10%, from about 0.025% to about 9%, from about 0.05% to about 5%, from about 0.1% to about 2.5%, from about 0.5% to about 2%, or from about 0.1% to about 1%, by weight of the composition. Non-limiting examples of anionic surfactants may include those described at US 2012/0082630 A1 at paragraphs 32, 33, 34, and 35. Non-limiting examples of zwitterionic or amphoteric surfactants may include those described at US 2012/0082630 A1 at paragraph 36; cationic surfactants may include those described at paragraphs 37 of the reference; and nonionic surfactants may include those described at paragraph 38 of the reference. Preferred surfactants include sodium lauryl sulfate, cocamidopropyl betaine, or mixtures thereof. In one aspect, the composition comprises from about 0.1% to about 5%, preferably about 0.1% to about 3%, preferably from about 0.3% to about 3%, preferably from about 1.2% to about 2.4%, preferably from about 1.2% to about 1.8%, preferably from about 1.5 % to about 1.8%, by weight of the composition, of an anionic surfactant, e.g. sodium lauryl sulfate (SLS).

### Colorant

The compositions herein may include a colorant. Titanium dioxide is one example of a colorant. Titanium dioxide is a white powder which adds opacity to the compositions. Colorant, e.g. titanium dioxide, generally can comprise from about 0.25% to about 5%, by weight of the composition.

### Flavorant

The compositions herein may include from about 0.001% to about 5%, preferably from about 0.01% to about 4%, preferably from about 0.1% to about 3%, preferably from about 0.5% to about 2%, preferably about 1% to about 1.5%, preferably about 0.5% to about 1%, by weight of the composition, of a flavorant. The term flavorant is used in the broadest sense to include flavor ingredients, sensates, or combinations thereof. Flavor ingredients may include those described in US 2012/0082630 A1 at paragraph 39; sensates may include those described at paragraphs 40 - 45, incorporated herein by reference. Excluded from the definition of flavorant is "sweetener" (as described above).

In one aspect, the composition of the present invention is free of an antimicrobial agent as described in US 6,296,834.

### Test Method: Assay for Measuring Biofilm Architecture & Thickness

The following assay is used an *in situ* plaque biofilm for inventive oral care compositions of the present invention in order to assess the biofilm Extracellular Polymeric Substances (EPS) matrix destabilization and thickness of the dental biofilm by measuring fluorescent light emitted from the labeled calcium & EPS within the biofilm.

The term "biofilm" refers to the layer(s) of cells attached to a surface. A biofilm can be a bacterial biofilm that includes both alive and growing microbe cells as well as dead microbe cells. The biofilm can be composed of one cell type or it may be composed of two or more cell types, for example, a biofilm complex that is a multispecies bacterial community. A specific type of biofilm is "dental biofilm" (also known as "plaque biofilm," used herein interchangeably) which is biofilm that typically forms on tooth surfaces in the human mouth). Bacteria in a plaque biofilm have significantly different physiological characteristics, e.g. increased resistance to detergents and antibiotics, making biofilm research highly important. A non-limiting list of oral bacterial species is described at US Pat. No. 6,309,835 B1, column 7, lines 12-30. These adherent microbe cells are frequently embedded within a self-produced matrix of extracellular polymeric substance ("EPS"). EPS are biopolymers of microbial origin in which biofilm microorganisms are embedded. J. Bacterial. 2007, 189(22):7945*.* Biofilm extracellular polymeric substance is a polymeric conglomeration generally composed of calcium, extracellular DNA, proteins, and polysaccharides.

Details of the assay are described below.

### (a) Substrate for Biofilm Growth

Hydroxyapatite ("HA") disks are used for *in situ* growth of biofilms. The HA disks are designed having three parallel grooves (*i.e.,* 200 µm wide; 200 µm deep for two sides' grooves; while 500 µm wide and 500 µm deep for the middle groove) in each disk. When attaching disks to subject's mouth, keep these grooves vertical, to mimic interproximal gap between teeth, which is the hard-to-clean area where plaque generally tends to accumulate. This model allows the collection of undisturbed plaque from the grooves. HA disks are manufactured by Shanghai Bei'erkang biomedicine limited company (Shanghai, China).

### (b) Wearing the Splint

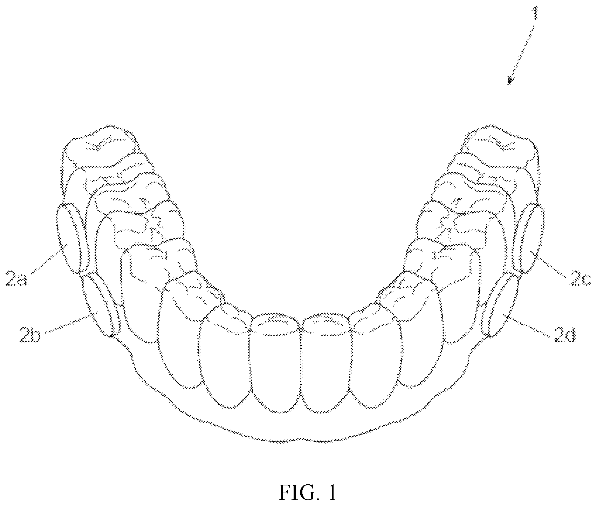

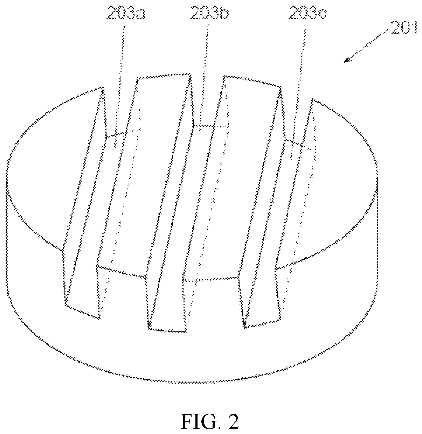

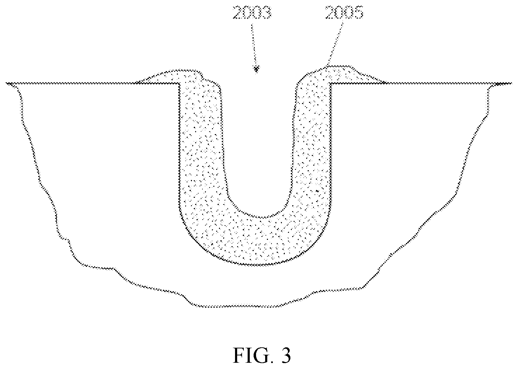

Human subjects wear the splint. Each subject wears up to 12 HA disks on the splint to ensure that, at least, 9 HA disks are available after 48 hours. A non-limiting example of such a splint and HA disks are shown in Figure 1. With reference to Figure 1, the device (1) holds a plurality of HA disks (2a-2d). In a specific example, and with reference to Figure 2, the HA disk (201) has three parallel grooves (203) (the two sides' grooves (203a and 203c) are 300 µm wide and 300 µm deep; while the middle grove (203b) (in between the two side grooves) is 500 µm wide and 500 µm deep). The middle groove is designed wider and deeper than the two sides' grooves so that the HA disk can be more easily separated into two identical half-disks for head-to-head comparison purposes. Figure 3 is a schematic of a cross-sectional view of the groove (2003) with biofilm (2005) therein. Further details of the HA disks are described in US2017/0056531 (e.g. paragraphs [0019] - [0020]).

Although not shown in Figure 3, the disks can be positioned such that the recede is in the inter-dental space between the teeth (since this location is prone to plaque (given the difficulty in cleaning, etc.)). The subjects withdraw the splint only during meals (the splint stored in an opaque container in humid conditions) and to perform oral hygiene procedures. Immediately thereafter, the splint is worn again. Subjects are asked to use a straw when drinking.

### (c) In-situ Biofilms Release from HA Desk

All HA disks are removed from the splint at 48 hours by tweezers. Tweezers are used to hold the edge of HA chips and transfer the HA disk to a 2 mL centrifuge tube containing PBS (phosphate buffered saline) solution. Tweezers are washed thoroughly (water; 75% alcohol; and then deionized water) before every disk transfer.

### (d) Preparation of Toothpaste Supernatant

15 grams of deionized water is added to 5 grams toothpaste (using any one of the Examples 1- 5). After stirring thoroughly, the mixture is centrifuge at 12,000 RPM for 20 minutes. The supernatant is prepared one day before usage and stored at 4 °C.

### (e) Confocal Laser Scanning Microscopy

After the HA disks are removed from the splint. The HA disks are used for *ex vivo* treatment by the different inventive and comparative compositions. After being treated with the subject supernatant and labeled with microbial fluorescent probe and stannous fluorescent probe (such as described in US2018/0072944A1; Shi *et al.*), the biofilms in the grooves are measured by confocal laser scanning microscopy ("CLSM") (as described below).

### (f) Disk Preparation

The HA disks are rinsed in PBS solution and each HA disk is divided into two halves by tweezers. Thereafter, each half-disk is placed into 500-1000 µL of PBS solution statically for 1 minute. Each disk is treated for two minutes by either PBS solution or toothpaste supernatant. Each disk is washed by holding each disk with tweezers, shaken for ten rounds of back and forth in 1 mL of PBS solution, and then this washing cycle is repeated. Then each disk is immersed into 500-1000 µL PBS solution statically for 5 minutes.

After being treated with PBS and/or the oral care composition (e.g., toothpaste) supernatant and labeled with specific fluorescent probes, the biofilm in the grooves is measured by confocal laser scanning microscopy (CLSM).

### (g) Fluorescence Probe Staining and Microscopy

"Ion fluorescent probe" means a fluorescent probe that specifically binds to one kind of ions and emit fluorescence at a certain wavelength. In recent years, significant emphasis has been placed on the development of new, highly selective fluorescent probes of ions because of their potential applications in biochemistry and environmental research. Many kinds of signaling mechanisms have been proposed and utilized for optical detection of ions, including photo-induced electron/energy transfer (PET), intramolecular charge transfer (ICT), fluorescence resonance energy transfer (FRET), and so on. Some of these fluorescent probes can also be applied in fluorescence bioimaging, which causes little cell damage and is highly sensitive with high-speed spatial analysis of living cells. Specifically, FRET imaging that affords simultaneous recording of two emission intensities at different wavelengths in the presence and absence of analytes has provided a facile method for visualizing complex biological processes at the molecular level. This technique appears to be suited to the study of physiological functions or pathogenesis of ions in biofilm and human body.

Fluorescence labeled calcium probes are molecules that exhibit an increase in fluorescence upon binding Ca2+. The biofilm is labeled with a calcium fluorescent probe. Examples of a calcium fluorescent probe suitable for labeling the biofilm may be any one or more of the following compounds:
(a) Fluo-3^{™}, AM^{™}, cell permeant fluorescence stains;
(b) Fluo-3^{™}, Pentapotassium Salt, cell impermeant fluorescence stains;
(c) Fluo-4^{™}, AM^{™}, cell permeant fluorescence stains;
(d) Fluo-4^{™}, Pentapotassium Salt, cell impermeant fluorescence stains;
(e) Fluo-4 Direct^{™} Calcium Assay Kit;
(f) Mag-Fluo-4^{™}, Tetrapotassium Salt, cell impermeant fluorescence stains; and
(g) Fluo-5F^{™}, AM^{™}, cell permeant fluorescence stains.
One or more of these probes may be available from ThermoFisher Scientific Company, Waltham, MA.

Fluo-3^{™} is used to image the spatial dynamics of Ca2+ signaling. Biofilm may be treated with the AM^{™} ester forms of calcium probes by adding the dissolved probe directly to biofilm. Fluo-3^{™}, AM^{™}, cell permeant fluorescent probes are used for intracellular and extracellular calcium staining using confocal microscopy, flow cytometry, and microplate screening applications (absorption/emission maxima ~506/526 nm). It is reported that the Concanavalin A^{™} (Con A), Alexa Fluor^{®} 594 Conjugate is a reliable alternative to stain EPS of biofilm. Alexa Fluor^{®} 594 conjugate of Con A exhibits the bright, red fluorescence of the Alexa Fluor^{®} 594 dye (absorption/emission maxima ~590/617 nm). Concanavalin A^{™}, Alexa Fluor^{®} 594 Conjugate selectively binds to α-mannopyranosyl and α-glucopyranosyl residues which are rich in EPS part of biofilm.

One specific example is Concanavalin A^{™}, Fluorescein Conjugate^{™}, wherein excitation is 494nm, and maximum light emission is detected at 518nm. These EPS fluorescent probes are widely available as well as the procedure details in how to use them to quantitatively determine the location and/or amount of EPS.

Examples of an EPS fluorescent probe suitable for labeling the biofilm may be any one of the following compounds:
(a) Molecular Probes^{™} Concanavalin A^{™} Alexa Fluor^{®} 350 Conjugate^{™};
(b) Molecular Probes^{™} Concanavalin A^{™} Alexa Fluor^{®} 488 Conjugate^{™};
(c) Molecular Probes^{™} Concanavalin A^{™} Alexa Fluor^{®} 594 Conjugate^{™};
(d) Molecular Probes^{™} Concanavalin A^{™} Alexa Fluor^{®} 633 Conjugate^{™};
(e) Molecular Probes^{™} Concanavalin A^{™} Alexa Fluor^{®} 647 Conjugate^{™};
(f) Molecular Probes^{™} Concanavalin A^{™} Fluorescein Conjugate^{™};
(g) Molecular Probes^{™} Concanavalin A^{™} Oregon Green^{®} 488 Conjugate^{™};
(h) Molecular Probes^{™} Concanavalin A^{™} tetramethylrhodamine Conjugate^{™};
(i) Molecular Probes^{™} Concanavalin A^{™} Texas Red^{®} Conjugate^{™}.
One or more of these probes may be available from ThermoFisher Scientific Company, Waltham, MA

After treatment and immersing, each half-disk specimen is stained with a dye mixture solution of the Fluo-3^{™}, AM^{™}, cell permeant fluorescent probe together with Concanavalin A^{™}, Alexa Fluor^{®} 594 Conjugate probe (containing 5uM Fluo-3^{™} + 5uM Con-A^{™}) for 30 minutes in the dark. After staining, each specimen is immersed into 500-1000 ul PBS solution statically for 2 minutes. The Fluo-3^{™}, AM^{™}/ Concanavalin A^{™}, Alexa Fluor^{®} 594 Conjugate dye stained samples, the following parameters are used: λex = 506 nm/590 nm, λem= 526/617 nm, 20X objective lens, and scanning from bottom of surface bacteria for 60 µm with step size= 3 µm.

### (h) Confocal Laser Scanning Microscopy

The Leica^{™} TCS SP8 AOBS spectral confocal microscope is used. The confocal system consists of a Leica^{™} DM6000B upright microscope and a Leica^{™} DMIRE2 inverted microscope. An upright stand is used for applications involving slide-mounted specimens; whereas the inverted stand, having a 37 °C incubation chamber and CO₂ enrichment accessories, provides for live cell applications. The microscopes share an exchangeable laser scan head and, in addition to their own electromotor-driven stages, a galvanometer-driven high precision Z-stage which facilitates rapid imaging in the focal (Z) plane. In addition to epifluorescence, the microscopes support a variety of transmitted light contrast methods including bright field, polarizing light and differential interference contrast, and are equipped with 5x, 20x, 40x, 63x (oil and dry) and 100x (oil) Leica^{™} objective lenses.

The laser scanning and detection system is described. The TCS SP8 AOBS confocal system is supplied with four lasers (one diode, one argon, and two helium neon lasers) thus allowing excitation of a broad range of fluorochromes within the UV, visible and far red ranges of the electromagnetic spectrum. The design of the laser scan head, which incorporates acousto-optical tunable filters ("AOTF"), an acousto-optical beam splitter ("AOBS") and four prism spectrophotometer detectors, permits simultaneous excitation and detection of three fluorochromes. The upright microscope also has a transmission light detector making it possible to overlay a transmitted light image upon a fluorescence recording.

Leica^{™} Confocal software LAS AF3.3.0 is used. The confocal is controlled via a standard Pentium PC equipped with dual monitors and running Leica^{™} Confocal Software. The Leica Confocal Software LAS AF3.3.0 (available from Leica Lasertechnik GmbH, Heidelberg, Germany) provides an interface for multi-dimensional image series acquisition, processing and analysis, that includes 3D reconstruction and measurement, physiological recording and analysis, time-lapse, fluorochrome co-localization, photo-bleaching techniques such as FRAP and FRET, spectral immixing, and multicolour restoration.

### (i) Image analysis

Ca:EPS; The Fluo-3^{™}/Con-A^{™} stained specimens, both fluorescence channels are chosen to quantify fluorescence intensity ratio of green pixels (Calcium) to red pixels (EPS) and Con-A^{™} fluorescence channel is chosen to measure the biofilm thickness. Whereby, six selected fields of Con-A^{™} fluorescence channel of each specimen are evaluated. These fields are considered as representative of the whole sample after the observer's general examination. The distance is measured from the surface of the biofilm to its base, measuring the thickness of the field, and subsequently the mean thickness of the biofilm of the corresponding specimen is calculated.

### EXAMPLES

Provided below are non-limited examples of the dentifrice compositions of the present invention, as well as comparative examples and a control example, in the form of toothpastes.

**TABLE 1: Compositions comprising calcium carbonate abrasive**

| | **Amount (% wt/wt)** | | | | | | |
|---|---|---|---|---|---|---|---|
| **Ingredients** | **Ex. 1 Control** | **Ex. 2 Comp.** | **Ex. 3 Comp.** | **Ex. 4** | **Ex. 5** | **Ex. 6** | **Ex. 7** |
| Sodium Monofluorophosphate | - | 1.10 | 1.10 | 1.10 | 1.10 | 1.10 | 1.10 |
| Sodium Carboxymethyl Cellulose | - | 1.30 | 1.30 | 1.30 | 1.10 | 1.30 | 1.30 |
| Thickening Silica | - | 6.00 | 3.00 | 3.00 | 1.50 | 3.00 | 3.00 |
| Glycine | - | - | - | 2.00 | 2.00 | 4.00 | 2.00 |
| Calcium Carbonate | - | 32.00 | 20.00 | 20.00 | 15.00 | 20.00 | 20.00 |
| Sodium Bicarbonate | - | - | 20.00 | 20.00 | 40.00 | 20.00 | 20.00 |
| Sodium Lauryl Sulfate (28% soln.) | - | 4.00 | 4.00 | 4.00 | 4.00 | 4.00 | 5.00 |
| Cocoamidopropyl betaine | - | - | - | - | - | - | 2.00 |
| Tetrasodium Pyrophosphate | - | 1.50 | 1.50 | 1.50 | 1.50 | 1.50 | 1.50 |
| Monosodium phosphate | - | 0.08 | 0.08 | 0.08 | 0.08 | 0.08 | 0.08 |
| Sodium Triphosphate | - | 0.42 | 0.42 | 0.42 | 0.42 | 0.42 | 0.42 |
| Benzyl Alcohol | - | 0.30 | 0.30 | 0.30 | 0.30 | 0.30 | 0.30 |
| Sodium Saccharin | - | 0.50 | 0.50 | 0.50 | 0.50 | 0.50 | 0.80 |
| Flavor/ sensate oils | - | 1.00 | 1.00 | 1.00 | 1.00 | 1.00 | 1.00 |
| Water & Minors (*e*.*g*., color soln.) | - | q.s. | q.s. | q.s. | qs | q.s. | qs |
| NaCl | 0.800 | - | - | - | - | - | - |
| KCL | 0.020 | - | - | - | - | - | - |
| Na2HPO4 | 0.142 | - | - | - | - | - | - |
| KH2PO4 | 0.024 | - | - | - | - | - | - |
| Water | 99.014 | - | - | - | - | - | - |
| Total | 100% | 100% | 100% | 100% | 100% | 100% | 100% |
| pH | 7.0 | 8.5 | 8.5 | 8.5 | 8.5 | 8.5 | 8.5 |

**TABLE 2: Compositions comprising silica abrasive**

| | **Amount (% wt/wt)** | | | | |
|---|---|---|---|---|---|
| **Ingredients** | **Ex. 8 Comp.** | **Ex. 9 Comp.** | **Ex. 10** | **Ex. 11** | **Ex. 12** |
| Sodium Fluoride | 0.32 | 0.32 | 0.32 | 0.32 | 0.32 |
| Sorbitol | 62.00 | - | 62.00 | - | - |
| Glycerin | - | 5.00 | - | 5.00 | 5.00 |
| Sodium Carboxymethyl Cellulose | 0.80 | 1.20 | 0.80 | 1.20 | 1.20 |
| Carbomer | 0.22 | - | 0.22 | - | - |
| Thickening Silica | - | 1.50 | - | 1.50 | 1.50 |
| Glycine | - | - | 2.00 | 2.00 | 2.00 |
| Cleaning Silica (Z119) | 16.00 | - | 16.00 | - | - |
| Cleaning Silica (Z109) | | 5.00 | | 5.00 | 5.00 |
| Sodium Bicarbonate | - | 40.00 | - | 40.00 | 40.00 |
| Sodium Lauryl Sulfate (28% soln.) | 5.00 | 5.00 | 5.00 | 5.00 | 5.00 |
| Cocoamidopropyl betaine | 2.00 | 2.00 | 2.00 | 2.00 | 2.00 |
| Tetrasodium Pyrophosphate | - | 1.50 | - | 1.50 | 1.50 |
| Sodium Hydroxide (50% aq) | - | 0.25 | - | 0.25 | 0.25 |
| Monosodium phosphate | 0.40 | - | 0.40 | - | - |
| Sodium Triphosphate | 0.93 | - | 0.93 | - | - |
| Sodium Saccharin | 0.30 | 0.50 | 0.50 | 0.50 | 0.80 |
| Flavor/ sensate oils | 1.10 | 1.10 | 1.10 | 1.10 | 1.10 |
| Water & Minors (*e.g*., color soln.) | q.s. | q.s. | - | q.s. | qs |
| Total | 100% | 100% | 100% | 100% | 100% |
| pH | 7.5 | 7.5 | 7.5 | 7.5 | 8.5 |

Data regarding Fluorescence Ratio of Ca/EPS and Biofilm Thickness, according to the test method described above, is provided for specific compositions as referenced in Tables 3 & 4 below.

**TABLE 3: Study #1 EPS/Calcium Ratio and Biofilm Thickness (with calcium carbonate abrasive)**

| **Product** | **Sodium Bicarbonate (%w/w)** | **Glycine (%w/w)** | **Fluorescence Ratio (Ca/EPS)** | **Biofilm Thickness (µm)** |
|---|---|---|---|---|
| Ex1 Control | 0 | 0 | 0.88 | 35.52 |
| Ex 2 Comp. | 0 | 0 | 0.64 | 23.32 |
| Ex 3 Comp. | 20 | 0 | 0.47 | 18.42 |
| Ex 4 | 20 | 2 | 0.22 | 12.90 |
| Ex 5 | 40 | 2 | 0.14 | 9.94 |
| Ex A¹ | 67 | 0 | 0.48 | 15.23 |

| | | | | |
|---|---|---|---|---|
| Ex A¹ Parodontax Classic ^{™} (LOT 14042610), having the ingredients: Sodium Bicarbonate, Aqua, Glycerin, Cocamidopropyl Betaine, Alcohol, Krameria Trianda Extract, Echinacea Purpurea Flower/Leaf/Stem Juice, Alcohol Denat., Xanthan Gum, Chamomilla Recutita Extract, Commiphora Myrrha Extract, Sodium Fluoride, Sodium Saccharin, Sodium Benzoate, Salvia Officinalis Oil, Mentha Piperita Oil, Mentha Arvensis Oil, Limonene, CI 77491 | | | | |

**TABLE 4. Study #2 EPS/Calcium Ratio and Biofilm Thickness (with silica abrasive)**

| **Product** | **Sodium Bicarbonate (%w/w)** | **Glycine (%w/w)** | **Fluorescence Ratio (Ca/EPS)** | **Biofilm Thickness (µm)** |
|---|---|---|---|---|
| Ex1 Control | 0 | 0 | 2.39 | 72.5 |
| Ex 8 Comp. | 0 | 0 | 2.05 | 35.4 |
| Ex 9 Comp. | 0 | 2 | 1.59 | 25.0 |
| Ex 10 | 40 | 0 | 1.56 | 23.8 |
| Ex 11 | 40 | 2 | 1.33 | 16.7 |
| Ex A¹ | 67 | 0 | 1.79 | 27.2 |
| Ex B¹ | 35 - 45 | 0 | 1.85 | 29.8 |

| | | | | |
|---|---|---|---|---|
| Ex A^{1:} Parodontax Original ^{™} (LOT 190796KWA), having the ingredients: Sodium Bicarbonate, Aqua, Glycerin, Alcohol, Cocamidopropyl Betaine, Mentha Oil, Mentha Piperita Oil, Xanthan Gum, Echinacea Purpurea Flower/Leaf/Stem Juice, Krameria Trianda Extract, Sodium Fluoride, Chamomilla Recutita Extract, Salvia Officinalis Oil, Commiphora Myrrha Extract, Limonene, Sodium Saccharin, Linalool, CI 77491. Ex B^{1:} Arm & Hammer Sensitive Pro Repair ^{™} (LOT FE9092D 2022/03), having the ingredients: Sodium Bicarbonate, PEG/PPG-38/8 Copolymer, Calcium Sulfate, PEG/PPG-116/66 Copolymer, Silica, Sodium Lauryl Sulfate, Aroma, Sodium Saccharin, Dipotassium Phosphate, PEG-8, Chrondus Crispus Powder, Sodium Fluoride, Limonene, CI 77891, | | | | |

Specific compositions as referenced in the Tables 1 and 2 along with controls were evaluated for saltiness taste, foam amount and in use dispersion using a method is a paired comparison study with randomised brushing order using 18 trained sensory panelists. Each assigned dentifrice is dispensed upon an Oral B Navigator brush and brushed for a controlled 2 minutes, with each panelist recording the sensorial attributes on the standard questionnaire with using a 0 to 5 grading score, with score of 0 being least sensorial preferred and 5 being the most sensorial preferred.

**TABLE 5. Sensory Grading**

| **Sensory Panel Grading** | **Ex A¹** | **Ex B¹** | **Ex. 4** | **Ex. 5** | **Ex. 7** | **Ex. 11** | **Ex. 12** |
|---|---|---|---|---|---|---|---|
| Saltiness Taste (during & post brushing) | 0 | 1 | 4 | 3 | 4 | 3 | 4 |
| Foam Amount (during brushing) | 1 | 2 | 3 | 3 | 4 | 4 | 4 |
| Dispersion (during brushing) | 3 | 1 | 4 | 4 | 4 | 5 | 5 |

| | | | | | | | |
|---|---|---|---|---|---|---|---|
| Saltiness Taste Attribute Grading Scale: Highly salty, unpleasant = 0 ; Moderate acceptable saltiness = 3; No saltiness = 5. Foam Amount Attribute Grading Scale: Poor, low foam = 0; Moderate foam = 3; Good, high foam = 5. Dispersion Attribute Grading Scale: Poor, hard to disperse= 0; Moderate disperse = 3; Good, easy to disperse foam = 5. | | | | | | | |

TABLES 3, 4 and 5 show the synergistic benefits of adding a suitable neutral amino acid, such as glycine, with sodium bicarbonate to reduce both Ca:EPS ratio and the biofilm thickness, whilst allowing for a more preferred sensorial experience.

Study #1, used calcium carbonate abrasive compositions, as shown in TABLE 1. Ex. 1, is a control composition that represents the salivary salts of a phosphate buffer solution. The use of Ex. 1 in Study # 1 TABLE 3, led to a final biofilm thickness of 35.52 µm, a Ca:EPS biofilm ratio of 0.88. Ex. 2 is a comparative dentifrice composition, as shown in TABLE 1, with absence of both sodium bicarbonate and glycine. The use of Ex. 2 led to a final biofilm thickness of 23.32 µm, a Ca:EPS biofilm ratio of 0.64. Ex. A¹ is a commercial dentifrice composition, with 67% sodium bicarbonate and no neutral amino acid (such as glycine). The use of Ex. A led to a final biofilm thickness of 15.23 µm, a Ca:EPS biofilm ratio of 0.48. The high sodium bicarbonate content of Ex. A led to a reduction in biofilm thickness and Ca:EPS ratio. However, this high sodium carbonate formulation of Ex. A, also led to poor sensorial grading for high saltiness taste (0) and poor/low foam (1) as shown in TABLE 5. The Ex. 3 is a comparative dentifrice composition, as shown in TABLE 1, with a lower sodium bicarbonate level and the absence of glycine. The use of Ex. 3 led to a final biofilm thickness of 18.42 µm, a Ca:EPS biofilm ratio of 0.47, similar to Ex. A¹, though had an improved sensorial experience grading with an acceptable saltiness taste (2) and moderate foaming (3). Ex. 4 is a dentifrice composition, as shown in TABLE 1, with the combination of both sodium bicarbonate and glycine. The use of Ex. 4 led to a final biofilm thickness of 12.90 µm, a Ca:EPS biofilm ratio of 0.22, and acceptable sensorial experience. Ex. 5 is a dentifrice composition, as shown in TABLE 1, with the combination of both mid-level of sodium bicarbonate and glycine. The use of Ex. 5 led to a final biofilm thickness of 9.94 µm, a Ca:EPS biofilm ratio of 0.14, and an acceptable sensorial experience. Ex. 7 is a dentifrice composition, as shown in TABLE 1, with the combination of both sodium bicarbonate and glycine and combination of mixed surfactants (sodium lauryl sulfate and cocamidopropyl betaine). The use of Ex. 7 led an improved sensorial experience, with acceptable saltiness (2) and good foaming (4).

Unexpectedly, the synergy in combining glycine with sodium bicarbonate, as in Ex. 4 with a calcium carbonate abrasive, led to a final biofilm thickness of 12.90 µm, and Ca:EPS of a 0.22. In other words, the addition of 2 wt.% of glycine with 20 wt.% sodium bicarbonate led to a 30% reduction in biofilm thickness and 54% reduction in Ca:EPS biofilm ratio vs. 20 wt.% sodium bicarbonate only as in Ex. 3. Additionally, Ex. 4 had improved sensorial experience vs. commercial high sodium bicarbonate dentifrices such as Ex. A. The addition of glycine at 2 wt.% with 40 wt.% sodium bicarbonate as in Ex. 5, led to a final biofilm thickness of 9.94 µm, which was a 35% reduction in biofilm thickness relative to the commercial high sodium bicarbonate dentifrice in Ex. A¹ (i.e. with no glycine), and had improved sensorial experience.

Further, Study #2 using silica based abrasive compositions, as shown in TABLE 2, also delivers similar unexpected synergy in combining glycine and sodium bicarbonate to reduce biofilm thickness, Ca:EPS ratio and have improved sensorial experience. The use of Ex. 1 in Study # 2 TABLE 4, led to a final biofilm thickness of 72.5 µm, a Ca:EPS biofilm ratio of 2.39 Ex. 8 is a comparative dentifrice composition, as shown in TABLE 2, with absence of both sodium bicarbonate and glycine. The use of Ex. 2 led to a final biofilm thickness of 35.4 µm, a Ca:EPS biofilm ratio of 2.05. Ex. A¹ is a commercial dentifrice composition, with 67% sodium bicarbonate and no neutral amino acid (such as glycine). The use of Ex. A led to a final biofilm thickness of 127.2 µm, a Ca:EPS biofilm ratio of 1.79. The high sodium bicarbonate content of Ex. A¹ led to a reduction in biofilm thickness and Ca:EPS ratio. However, this high sodium carbonate formulation of Ex. A¹, also led to poor sensorial grading for high saltiness taste (0), poor/low foam (1) and poor dispersion (3) as shown in TABLE 5. Ex. B¹ is a commercial dentifrice composition, with circa 35% to 45% sodium bicarbonate and no neutral amino acid (such as glycine) formulated as an anhydrous composition, led to a final biofilm thickness of 29.8 µm, a Ca:EPS biofilm ratio of 1.85. However, this anhydrous composition of Ex. B¹, also led to poor sensorial grading for saltiness taste (1), poor/low foam (2) and dispersion (1) as shown in TABLE 5. Ex. 11 is a dentifrice composition, as shown in TABLE 2, with the combination of both sodium bicarbonate and glycine. The use of Ex. 11 led to a final biofilm thickness of 16.7 µm, a Ca:EPS biofilm ratio of 1.33, and acceptable sensorial experience, with acceptable saltiness (3), good foaming (4) and good dispersion (5).

Unexpectedly, the synergy in combining glycine with sodium bicarbonate with a silica abrasive , as in Ex. 11, led to a final biofilm thickness of 16.7 µm, and Ca:EPS of a 1.56. In other words, the addition of 2 wt.% of glycine with 40 wt.% sodium bicarbonate led to a 30% reduction in biofilm thickness and 15% reduction in Ca:EPS biofilm ratio vs. 40 wt.% sodium bicarbonate only as in Ex. 10. Additionally, Ex. 11 had improved sensorial experience vs. commercial high sodium bicarbonate dentifrices such as Ex. A¹ and Ex B¹. The addition of glycine at 2 wt.% with 40 wt.% sodium bicarbonate as in Ex. 11, led to a final biofilm thickness reduction of 38% and 44% compared to the sodium bicarbonate dentifrices in Ex. A¹ & Ex. B¹ respectively (i.e. with no glycine), and had improved sensorial experience.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A dentifrice composition comprising:
(a) from about 15% to 55%, by weight of the composition, of water:
(b) from 20% to 40%, by weight of the composition, of bicarbonate salt;
(c) from 0.01% to 10%, by weight of the composition, of neutral amino acid: and
(d) from 0.0025% to 2%, by weight of the composition, of a fluoride ion source; and
wherein the neutral amino acid is not complexed with zinc.

2. The dentifrice composition of claim 1, wherein the composition further comprises from 0% to 10%, preferably from 0% to 5%, by weight of the composition, of a humectant, wherein the humectant is a polyol, preferably selected from sorbitol, glycerol, or mixtures thereof.

3. The dentifrice composition of any one of the preceding claims, wherein the neutral amino acid is selected from the group consisting of alanine, aminobutyrate, asparagine, cysteine, cystine, glutamine, glycine, hydroxyproline, isoleucine, leucine, methionine, phenylalanine, proline, serine, taurine, threonine, tryptophan, tyrosine, valine, salts thereof, and mixtures thereof; preferably selected from the group consisting of glycine, asparagine, glutamine, salts thereof, and mixtures thereof; and wherein preferably the neutral amino acid is present in the amount of from 0.05% to 5%, more preferably from 1% to 3%, by weight of the composition.

4. The dentifrice composition of any one of the preceding claims, wherein the neutral amino acid is glycine or a salt thereof.

5. The dentifrice composition of any one of the preceding claims, wherein the composition further comprises from 20% to 50%, and preferably 25% to 45% by weight of the composition of water.

6. The dentifrice composition of any one of the preceding claims, wherein the dentifrice composition comprises from 0.01% to 10%, preferably from 1% to 5%, by weight of the composition, of silica abrasive.

7. The dentifrice composition of any one of the preceding claims, wherein the composition has a pH greater than 7.3 .

8. The dentifrice composition of any one of the preceding claims, wherein the bicarbonate salt is sodium bicarbonate.

9. The dentifrice composition of any one of the preceding claims, wherein the composition comprises from 5% to 50%, preferably from 10% to 50%, more preferably from 15% to 40%, by weight of the composition, of a calcium-containing abrasive, and preferably wherein the calcium-containing abrasive is calcium carbonate.

10. The dentifrice composition of any one of the preceding claims, wherein the dentifrice composition comprises calcium-containing abrasive and silica abrasive.

11. The dentifrice composition of any one of the preceding claims, wherein the dentifrice composition comprises a thickening silica.

12. The dentifrice composition of any one of the preceding claims, wherein the dentifrice composition comprises a thickening polymer, wherein the thickening polymer is selected from group consisting of a carboxymethyl cellulose, a linear sulfated polysaccharide, a natural gum, and mixtures thereof.

13. The dentifrice composition any one of the preceding claims, wherein the fluoride ion source is either sodium monofluorophosphate, amine fluoride or sodium fluoride and combinations thereof.

14. The dentifrice composition of any one of the preceding claims, wherein the dentifrice composition comprises from 0.5% to 1.5%, by weight of the composition, of the fluoride ion source.

15. The dentifrice composition of any one of the preceding claims, wherein the neutral amino acid is uncomplexed.

16. The dentifrice composition of any one of the preceding claims, wherein the dentifrice composition is a single phase toothpaste.

## Patentansprüche

1. Zahncremezusammensetzung, umfassend:
(a) von zu 15 Gew.-% bis 55 Gew.-% der Zusammensetzung Wasser;
(b) von zu 20 Gew.-% bis 40 Gew.-% der Zusammensetzung Bicarbonatsalz;
(c) von zu 0,01 Gew.-% bis 10 Gew.-% der Zusammensetzung eine neutrale Aminosäure; und
(d) von zu 0,0025 Gew.-% bis 2 Gew.-% der Zusammensetzung eine Fluoridionenquelle; und
wobei die neutrale Aminosäure nicht mit Zink komplexiert ist.

2. Zahncremezusammensetzung nach Anspruch 1, wobei die Zusammensetzung ferner von zu 0 Gew.-% bis 10 Gew.-%, vorzugsweise von zu 0 Gew.-% bis 5 Gew.-%, der Zusammensetzung ein Feuchthaltemittel umfasst, wobei das Feuchthaltemittel ein Polyol ist, das vorzugsweise ausgewählt ist aus Sorbit, Glycerin oder Mischungen davon.

3. Zahncremezusammensetzung nach einem der vorstehenden Ansprüche, wobei die neutrale Aminosäure ausgewählt ist aus der Gruppe bestehend aus Alanin, Aminobutyrat, Asparagin, Cystein, Cystin, Glutamin, Glycin, Hydroxyprolin, Isoleucin, Leucin, Methionin, Phenylalanin, Prolin, Serin, Taurin, Threonin, Tryptophan, Tyrosin, Valin, Salzen davon und Mischungen davon; vorzugsweise ausgewählt ist aus der Gruppe bestehend aus Glycin, Asparagin, Glutamin, Salzen davon und Mischungen davon; und wobei vorzugsweise die neutrale Aminosäure in der Menge von zu 0,05 Gew.-% bis 5 Gew.-%, mehr bevorzugt von zu 1 Gew.-% bis 3 Gew.-%, der Zusammensetzung gegenwärtig ist.

4. Zahncremezusammensetzung nach einem der vorstehenden Ansprüche, wobei die neutrale Aminosäure Glycin oder ein Salz davon ist.

5. Zahncremezusammensetzung nach einem der vorstehenden Ansprüche, wobei die Zusammensetzung ferner von zu 20 Gew.-% bis 50 Gew.-%, und vorzugsweise von zu 25 Gew.-% bis 45 Gew.-%, der Zusammensetzung Wasser umfasst.

6. Zahncremezusammensetzung nach einem der vorstehenden Ansprüche, wobei die Zahncremezusammensetzung von zu 0,01 Gew.-% bis 10 Gew.-%, vorzugsweise von zu 1 Gew.-% bis 5 Gew.-%, der Zusammensetzung ein Silicaschleifmittel umfasst.

7. Zahncremezusammensetzung nach einem der vorstehenden Ansprüche, wobei die Zusammensetzung einen pH-Wert größer als 7,3 aufweist.

8. Zahncremezusammensetzung nach einem der vorstehenden Ansprüche, wobei das Bicarbonatsalz Natriumbicarbonat ist.

9. Zahncremezusammensetzung nach einem der vorstehenden Ansprüche, wobei die Zusammensetzung von zu 5 Gew.-% bis 50 Gew.-%, vorzugsweise von zu 10 Gew.-% bis 50 Gew.-%, mehr bevorzugt von zu 15 Gew.-% bis 40 Gew.-%, der Zusammensetzung ein calciumhaltiges Schleifmittel umfasst, und wobei vorzugsweise das calciumhaltige Schleifmittel Calciumcarbonat ist.

10. Zahncremezusammensetzung nach einem der vorstehenden Ansprüche, wobei die Zahncremezusammensetzung calciumhaltiges Schleifmittel und Silicaschleifmittel umfasst.

11. Zahncremezusammensetzung nach einem der vorstehenden Ansprüche, wobei die Zahncremezusammensetzung ein Verdickungssilica umfasst.

12. Zahncremezusammensetzung nach einem der vorstehenden Ansprüche, wobei die Zahncremezusammensetzung ein Verdickungspolymer umfasst, wobei das Verdickungspolymer ausgewählt ist aus der Gruppe bestehend aus Carboxymethylzellulose, einem linearen sulfatierten Polysaccharid, einem Pflanzengummi und Mischungen davon.

13. Zahncremezusammensetzung nach einem der vorstehenden Ansprüche, wobei die Fluoridionenquelle entweder Natriummonofluorphosphat, Aminfluorid oder Natriumfluorid und Kombinationen davon ist.

14. Zahncremezusammensetzung nach einem der vorstehenden Ansprüche, wobei die Zahncremezusammensetzung von zu 0,5 Gew.-% bis 1,5 Gew.-% der Zusammensetzung die Fluoridionenquelle umfasst.

15. Zahncremezusammensetzung nach einem der vorstehenden Ansprüche, wobei die neutrale Aminosäure nicht komplexiert ist.

16. Zahncremezusammensetzung nach einem der vorstehenden Ansprüche, wobei die Zahncremezusammensetzung eine einphasige Zahnpasta ist.

## Revendications

1. Composition dentifrice comprenant :
(a) de 15 % à 55 %, en poids de la composition, d'eau ;
(b) de 20 % à 40 %, en poids de la composition, de sels de carbonate ;
(c) de 0,01 % à 10 % en poids de la composition, d'acide aminé neutre ; et
(d) de 0,0025 % à 2 %, en poids de la composition, d'une source d'ions fluorure ; et
dans laquelle l'acide aminé neutre n'est pas complexé avec le zinc.

2. Composition de dentifrice selon la revendication 1, dans laquelle la composition comprend en outre de 0 % à 10 %, de préférence de 0 % à 5 %, en poids de la composition, d'un humectant, dans laquelle l'humectant est un polyol, de préférence choisi parmi le sorbitol, le glycérol, ou leurs mélanges.

3. Composition de dentifrice selon l'une quelconque des revendications précédentes, dans laquelle l'acide aminé neutre est choisi dans le groupe constitué par l'alanine, l'aminobutyrate, l'asparagine, la cystéine, la cystine, la glutamine, la glycine, l'hydroxyproline, l'isoleucine, la leucine, la méthionine, la phénylalanine, la proline, la sérine, la taurine, la thréonine, le tryptophane, la tyrosine, la valine, les sels de ces acides et leurs mélanges ; de préférence choisi dans le groupe constitué par la glycine, l'asparagine, la glutamine, leurs sels et leurs mélanges ; et dans laquelle, de préférence, l'acide aminé neutre est présent à raison de 0,05 % à 5 %, plus préférablement de 1 % à 3 %, en poids de la composition.

4. Composition de dentifrice selon l'une quelconque des revendications précédentes, dans laquelle l'acide aminé neutre est la glycine ou un de ses sels.

5. Composition de dentifrice selon l'une quelconque des revendications précédentes, dans laquelle la composition comprend en outre de 20 % à 50 %, et de préférence de 25 % à 45 %, en poids de la composition d'eau.

6. Composition de dentifrice selon l'une quelconque des revendications précédentes, dans laquelle la composition comprend de 0,01 % à 10 %, de préférence de 1 % à 5 %, en poids de la composition, de silice abrasive.

7. Composition de dentifrice selon l'une quelconque des revendications précédentes, dans laquelle la composition a un pH supérieur à 7,3.

8. Composition de dentifrice selon l'une quelconque des revendications précédentes, dans laquelle le sel de bicarbonate est du bicarbonate de sodium.

9. Composition de dentifrice selon l'une quelconque des revendications précédentes, dans laquelle la composition comprend de 5 % à 50 %, de préférence de 10 % à 50 %, plus préférablement de 15 % à 40 %, en poids de la composition, d'un abrasif contenant du calcium, et de préférence dans laquelle l'abrasif contenant du calcium est du carbonate de calcium.

10. Composition de dentifrice selon l'une quelconque des revendications précédentes, dans laquelle la composition de dentifrice comprend un abrasif contenant du calcium et un abrasif de silice.

11. Composition dentifrice selon l'une quelconque des revendications précédentes, dans laquelle la composition de dentifrice comprend une silice épaississant.

12. Composition de dentifrice selon l'une quelconque des revendications précédentes, dans laquelle la composition de dentifrice comprend un polymère épaississant, dans laquelle le polymère épaississant est choisi dans le groupe constitué d'une carboxyméthylcellulose, d'un polysaccharide sulfaté linéaire, d'une gomme naturelle, et de leurs mélanges.

13. Composition de dentifrice selon l'une quelconque des revendications précédentes, dans laquelle la source d'ions fluorure est soit du monofluorophosphate de sodium, du fluorure d'amines ou du fluorure de sodium et leurs combinaisons.

14. Composition de dentifrice selon l'une quelconque des revendications précédentes, dans laquelle la composition comprend de 0,5 % à 1,5 %, en poids de la composition, de la source d'ions fluorure.

15. Composition de dentifrice selon l'une quelconque des revendications précédentes, dans laquelle l'acide aminé neutre est non complexée.

16. Composition de dentifrice selon l'une quelconque des revendications précédentes, dans laquelle la composition de dentifrice est une composition monophasée.
